(19) Europäisches Patentamt
European Patent Office
Office européen des brevets

(11) Veröffentlichungsnummer : **0 024 664**
**B1**

(12) **EUROPÄISCHE PATENTSCHRIFT**

(45) Veröffentlichungstag der Patentschrift : **16.02.83**

(51) Int. Cl.³ : **C 07 C103/52**

(21) Anmeldenummer : **80104880.2**

(22) Anmeldetag : **16.08.80**

# (54) Neue Peptidamide und Verfahren zu ihrer Herstellung.

(30) Priorität : **22.08.79 DE 2933947**

(43) Veröffentlichungstag der Anmeldung : **11.03.81 Patentblatt 81/10**

(45) Bekanntmachung des Hinweises auf die Patenterteilung : **16.02.83 Patentblatt 83/07**

(84) Benannte Vertragsstaaten : **AT BE CH DE FR GB IT LI NL SE**

(56) Entgegenhaltungen :
**EP A 0 005 248**
**WO A 80/00022**
**DE A 2 715 803**
**DE A 2 732 451**
**DE A 2 739 440**

(73) Patentinhaber : **HOECHST AKTIENGESELLSCHAFT**
**Postfach 80 03 20**
**D-6230 Frankfurt/Main 80 (DE)**

(72) Erfinder : **Teetz, Volker, Dr.**
**An der Tann 20**
**D-6238 Hofheim am Taunus (DE)**
Erfinder : **Geiger, Rolf, Dr.**
**Heinrich-Bleicher-Strasse 33**
**D-6000 Frankfurt am Main 50 (DE)**
Erfinder : **Alpermann, Hans Georg, Dr.**
**Am Eichkopf 10**
**D-6240 Königstein/Taunus (DE)**
Erfinder : **Bickel, Martin, Dr.**
**Beethovenstrasse 40**
**D-6000 Frankfurt am Main (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 024 664 B1

Neue Peptidamide und Verfahren zu ihrer Herstellung

Gegenstand der Erfindung sinJ Peptidamide der Formel I

H—Tyr—D—Lys(For)—Gly—X (I)

in der bedeutet
For Formyl,
X Alkylamino,
Dehydro-Phe- oder Phe-alkylamido mit bis zu 6 C-Atomen im Alkylrest, der durch Hydroxy und/oder Phenyl substituiert sein kann,
Phe-cycloalkylamid oder -cycloalkylenamid mit bis zu 8 C-Atomen im Cycloalkyl bzw. Cycloalkylen, wobei 1-2-$CH_2$-Gruppen durch —NH—, —O—, —S— oder —CO— ersetzt sein können,
Phe-alkylen-cycloalkylamid mit 5-6 Ring-C-Atomen, das durch Carbonamido, N-Alkylcarbonamido oder Alkyl substituiert sein kann und in dem ein C-Atom durch N ersetzt sein kann,
Phe-endo- oder exo-norbornylamid oder Phe-thiazolamid oder -thiazolidincarbonsäureamid, das durch 1-4 Methylgruppen substituiert sein kann.

Gegenstand der Erfindung ist ferner ein Verfahren zur Herstellung der Verbindungen der Formel I, das dadurch gekennzeichnet ist, daß man ein Peptidderivat der Formel II

Y—Tyr—D—Lys(For)—Gly—OH (II)

in der
Y eine Schutzgruppe, vorzugsweise Benzyloxycarbonyl oder tert.-Butyloxycarbonyl bedeutet, mit einem Amin XH kondensiert oder ein Peptidderivat der Formel III

Y—Tyr—D—Lys(For)—Gly—Phe—OH (III)

mit einem um den Phenylalaninrest (Phe) verkürzten Amin XH umsetzt und anschließend die Schutzgruppe Y abspaltet.

Für die Synthese der erfindungsgemäßen Verbindungen eignen sich die allgemein bekannten Methoden der Peptidchemie (vgl. Houben-Weyl, Methoden der Organischen Chemie, Band 15). Bevorzugt wird die Methode der Aktiven Ester angewandt, wobei als Katalysator 1-Hydroxybenzotriazol zugesetzt werden kann (Chem. Ber. *106* (1973), Seite 3626). Ebenfalls bevorzugt ist die Carbodiimidmethode unter Zusatz einer Aktivester bildenden Komponente, bevorzugt 1-Hydroxybenzotriazol.

Die Umsetzung wird in einem hierfür geeigneten und bekannten Lösungsmittel ausgeführt. Als bevorzugte Lösungsmittel dienen Dialkylamide wie Dimethylformamid, Dimethylacetamid oder N-Methylpyrrolidon. Die Konzentration der Reaktionspartner entspricht der bei derartigen Synthesen üblichen.

Die Umsetzung wird bei einer Temperatur durchgeführt, bei der das angewandte Lösungsmittel in flüssiger Form vorliegt und bei der die Reaktionspartner keinen irreversiblen Schaden erleiden. Die bevorzugte Reaktionstemperatur liegt zwischen etwa 0° und etwa 40-60 °C, wobei Raumtemperatur besonders bevorzugt ist.

Als N-Schutzgruppen im Sinne der Erfindung kommen alle protonensolvolytisch leicht abspaltbaren sowie photosensitiven oder β-eliminierbaren Reste beispielsweise solche vom Benzyloxycarbonyl-, tert.Alkyl-, tert.Aralkyl- und Alkyloxycarbonyl-Typ in Frage, ferner Schutzgruppen wie Trityl-, 2-Nitrophenylsulfenyl-, 1-Methyl-2-acyl-vinyl und Trifluoracetyl. Als Schutzgruppe sind ebenfalls enzymatisch abspaltbare N-geschützte Aminosäuren wie Acylarginin verwendbar.

Diese Schutzgruppen sind im vorher erwähnten Band des Handbuches von Houben-Weyl ausführlich beschrieben.

Ungeeignet als N-Schutzgruppen im Sinne der Erfindung sind neben dem Formylrest insbesondere solche Gruppen, die unter Bedingungen eleminiert werden müssen, die auch den Formylrest entfernen. Hierzu gehört z. B. Phthaloyl, das wie Formyl mit Hydrazin abspaltbar ist. Ungeeignet sind auch Schutzgruppen, die nur mit Bromycan oder mit einem oxidierenden Agens abspaltbar sind. Das gilt insbesondere für Verbindungen der Formel I, in denen X für einen schwefelhaltigen Substituenten steht. Auch der Tosylrest ist wegen seiner schweren Abspaltbarkeit ungeeignet.

In Tabelle 1 sind einige charakteristische Verbindungen gemäß der Erfindung aufgeführt.

(Siehe die Tabelle 1, Seite 3)

Tabelle 1 : Charakterisierung erfindungsgemäßer Verbindungen

| X | Rf[1)] | Schmp. | charakterisiert durch[2)] | $[\alpha]_D$ (C=1,DMF) |
|---|---|---|---|---|
| Phe-NH-$(CH_2)_5$-$CH_3$ | 0.57 | | AA, CHN | + 17° |
| Phe-Pro-NH-$(CH_2)_5$-$CH_3$ | 0.53 | | AA | - 12° |
| Phe-Pro-$NH_2$ | 0.39 | | AA, CHN | - 20.8° |
| $N^{\alpha}$-Z-Verbindung | | 125-7° | CHN | |
| Phe-NH 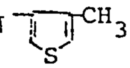 | 0.58 | | AA, CHN | - 5.4° |
| $N^{\alpha}$-Boc-Verbindung | | 161-2° | CHN | |
| Phe- 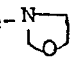 | 0.48 | | AA | |
| $N^{\alpha}$-Boc-Verbindung | | | CHN | |
| Phe-N 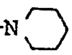 | 0.49 | | AA | +8.3° |
| $N^{\alpha}$-Boc-Verbindung | | 84° | CHN | |
| Phe-N 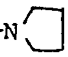 | 0.45 | | AA | +19.5° |
| $N^{\alpha}$-Boc-Verbindung | | | CHN | |
| Phe-N 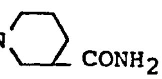 | 0.40 | | AA | +7.4° |
| $N^{\alpha}$-Boc-Verbindung | | 112-3° | CHN | |
| Phe-NH 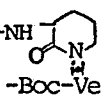 | 0.37 | | AA | -1.1° |
| $N^{\alpha}$-Boc-Verbindung | | 138° | CHN | |
| Phe-N 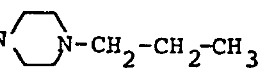 | 0.20 | | AA | +2.6° |
| $N^{\alpha}$-Boc-Verbindung | | 198-200° | CHN | |
| Phe-N 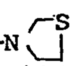 | 0.43 | | AA, CHN | +20° |
| Phe-N 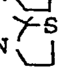 | 0.51 | | AA, CHN | +25° |
| Phe-N 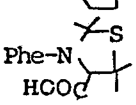 | 0.15 | | AA, CHN | -10,5° |
| Phe-NH- 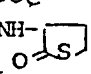 | 0.45 | | AA | +7.7° |
| $N^{\alpha}$-Boc-Verbindung | | 167-8° | CHN | |
| Phe-endo-norbornylamid | 0.57 | | AA | +3.4° |
| $N^{\alpha}$-Boc-Verbindung | | 119° | CHN | |
| Phe-exo-norbornylamid | 0.58 | | AA | +4.8° |
| $N^{\alpha}$-Boc-Verbindung | | 108° | CHN | |
| Phe-NH-$CH_2$- 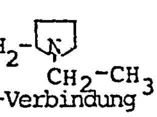 | 0.17 | | AA | +3.8° |
| $N^{\alpha}$-Boc-Verbindung | | 86° | CHN | |
| Phe-NH-CH($CH_3$)-$CH_2$-OH | 0.44 | | AA | +10.0° |

Tabelle 1 (Fortsetzung)

| X | Rf 1) | Schmp. | charakterisiert durch 2) | [α]$_D$ (C=1,DMF) |
|---|---|---|---|---|
| N$^{\alpha}$-Boc-Verbindung | | 145-8° | CHN | |
| Phe-NH-$CH_2$-CH(OH)-$CH_3$ | 0.44 | | AA | +6.1° |
| N$^{\alpha}$-Boc-Verbindung | | 146°(Z) | CHN | |
| 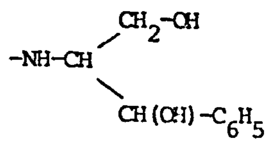 | 0.50 | | AA | +52.6° |
| N$^{\alpha}$-Boc-Verbindung | | | CHN | +28.8° |
| 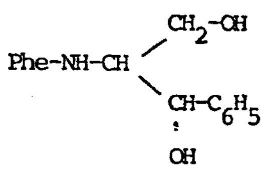 | 0.49 | | AA | +23.5° |
| N$^{\alpha}$-Boc-Verbindung | | | CHN | +12.8° |

1) DC im System n-Butanol/Essigsäure/Wasser (3 : 1 : 1)
Standard : Tyr—D—Lys(For)—Gly—Phe—OH : Rf = 0,42
2) AA = Aminosäureanalyse
CHN = Elementaranalyse von C, H und N

Die erfindungsgemäßen Verbindungen sind in der Lage, Morphinrezeptoren zu besetzen. Sie wirken deshalb analgetisch und unterdrücken die Motilität des Meerschweinchen-Ileums.

Nach intravenöser Gabe sind sie mit 1-100 mg/kg, bevorzugt 1-10 mg/kg, wirksam. Überraschenderweise rufen sie am Hund schon mit 0,01 mg/kg eine außerordentlich starke Steigerung der Darmmotilität hervor. Sie sind deshalb bei postoperativer Darmatonie einsetzbar. Zur Anwendung gelangen sie bevorzugt in isotonischer wäßriger Lösung von pH 4-6 unter intravenöser oder intramuskulärer Injektion oder in einer solchen Lösung auf intranasalem Wege.

Tabelle 2 gibt über die biologische Wirkung einer charakteristischen erfindungsgemäßen Verbindung (A) im Vergleich zu einem aus Biochem. Biophys. Res. Commun. *84* (1978), S. 1045 bekannten, sehr stark wirksamen Peptidderivat (B) Auskunft (ED + 100 % : Verdoppelung der Reaktionszeit, $ED_{50}$ : Reduktion der Reizantwort um 50 %).

Tabelle 2 : Biologische Wirkung der erfindungsgemäßen Verbindungen am Beispiel des Peptidderivats nach Anspruch 3

| | Peptidderivat | Analgesie Maus ED+100 ≙ mg/kg | Meerschweinchen-Ileum $ED_{50}$ g/ml | Darmmotilität (Hund) mit 0,01 mg/kg |
|---|---|---|---|---|
| A | Tyr-D-Lys(For)-Gly-Phe-NH 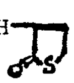 | 2 | $3.10^{-10}$ | wirksam |
| B | Tyr-D-Met-Gly-Phe-Pro-$NH_2$ | 2 | $3.10^{-9}$ | |

Man erkennt, daß die erfindungsgemäße Verbindung am Meerschweinden-Ileum zehnmal stärker wirkt als die Vergleichssubstanz, die hinsichtlich der analgetischen Wirkung ebenso aktiv ist. Es ist somit ein deutlicher Wirkungssplit vorhanden, der durch den neuartigen Effekt auf die Darmmotilität des Hundes in kleinster Dosierung noch unterstrichen wird.

Die nachfolgenden Beispiele beschreiben die Synthese einiger charakteristischer Verbindungen. In analoger Weise werden die übrigen erfindungsgemäßen Verbindungen erhalten.

Beispiel 1

L-Tyrosyl-D-N$^{\varepsilon}$-formyllysyl-glycyl-L-phenylalanyl-homocysteinthiolacton-trifluoracetat

a) Benzyloxycarbonyl-glycyl-phenylalanin

16.5 g Phenylalanin werden in 150 ml Dimethylformamid (DMF) suspendiert und mit 13,5 g 1-Hydroxybenzotriazol, 38.8 g Benzyloxycarbonylglycin-trichlorphenylester sowie 12.8 ml N-Ethylmorpholin 8 Stunden bei Raumtemperatur gerührt. Man engt im Vakuum ein, nimmt in 1 l Essigester auf und schüttelt mehrfach mit je 200 ml 2N Citronensäure und gesättigter Natriumchloridlösung aus. Die organische Phase wird über Natriumsulfat getrocknet und im Vakuum eingeengt. Der Rückstand kristallisiert aus Essigester/Diisopropyläther.
Ausbeute : 23,5 g = 66 %,
Schmp. 125-126° $[\alpha]_D^{22} = +28.1$ (C = 1, Methanol)

b) Glycyl-phenylalanin-hydrochlorid

23 g Benzyloxycarbonyl-glycyl-phenylalanin werden in 250 ml eines Methanol/Dimethylformamid-Gemisches (1 : 1) gelöst und unter Zusatz von 1 g Palladium/Kohle (5 % Pd) (Pd/C) katalytisch hydriert. Der pH-Wert wird durch Zugabe von methanolischer Salzsäure bei pH 4.5 gehalten. Nach Entfernung des Katalysators wird das Filtrat im Vakuum eingeengt und der Rückstand aus Methanol durch Zusatz von Äther gefällt.
Ausbeute : 15.4 g = 93 %,
Schmp. 264-265° $[\alpha]_D^{22} = +30.7°$ (c = 1, 2n HCl)

c) Benzyloxycarbonyl-D-N$^{\varepsilon}$-formyllysyl-glycyl-phenylalanin

15 g Glycyl-phenylalanin-hydrochlorid werden in 200 ml DMF suspendiert und mit 7.8 g 1-Hydroxybenzotriazol und 28.3 g Benzyloxycarbonyl-D-N$^{\varepsilon}$-formyllysin-trichlorphenylester unter Zusatz von 7.5 ml N-Ethylmorpholin 8 Stunden bei Raumtemperatur gerührt. Nach Einengen im Vakuum wird in Essigester aufgenommen und mit 2n Citronensäure und NaCl-Lösung ausgeschüttelt. Die über Natriumsulfat getrocknete organische Phase wird etwas eingeengt und das Produkt durch Zugabe von Äther kristallisiert.
Ausbeute : 22.4 g = 75 %,
Schmp. 150-152°, $[\alpha]_D^{22} = +7°$ (c = 1, Methano)

d) D-N$^{\varepsilon}$-Formyllysyl-glycyl-phenylalanin

22 g Benzyloxycarbonyl-D-N$^{\varepsilon}$-formyllysyl-glycyl-phenylalanin werden in DMF/Wasser (1 : 1) gelöst, mit 500 mg Pd/C versetzt und katalytisch hydriert. Das Filtrat wird im Vakuum eingeengt und aus einer kleinen Menge wässrigem Methanol kristallisiert.
Ausbeute 13.2 g = 81 %,
Schmp. 180-181°, $[\alpha]_D^{22} = -2.4$ (c = 1, 2n HCl)

e) tert. Butyloxycarbonyl-O-tert.butyl-tyrosyl-D-N$^{\varepsilon}$-formyllysyl-glycyl-phenylalanin

13 g D-N$^{\varepsilon}$-formyllysyl-glycyl-phenylalanin werden in 250 ml DMF suspendiert und mit 4.6 g 1-Hydroxybenzotriazol, 14.9 g tert.Butyloxycarbonyl-O-tert-butyltyrosin-N-hydroxysuccinimidester sowie 4.4 ml N-Ethylmorpholin 24 Stunden bei Raumtemperatur gerührt. Nach Einengen im Vakuum wird in Essigester aufgenommen. Nach dem Ausschütteln des in Essigester gelösten Reaktionsgemisches mit 2n Citronensäure und wässriger Natriumchloridlösung wird die Substanz aus Essigester/Äther kristallisiert.
Ausbeute : 17 g = 71 %,
Schmp. 123-124°, $[\alpha]_D^{22} = +40.3°$ (c = 1, Methanol)

f) tert.Butyloxycarbonyl-O-tert-butyl-tyrosyl-D-N$^{\varepsilon}$-formyllysyl-glycyl-phenylalanyl-homocysteinthiolacton

3.49 g BOC—Tyr(Bu$^t$)—D—Lys(For)—Gly—Phe—OH werden mit 675 mg 1-Hydroxybenzotriazol und 768 mg DL-Homocysteinthiolacton-hydrochlorid in 20 ml DMF gelöst. Man kühlt auf 0 °C und fügt 640 µl N-Ethylmorpholin und 1,04 g Dicyclohexylcarbodiimid zu. Nach einer Reaktionszeit von 2 Stunden bei 0 °C hält man noch 4 Stunden bei Raumtemperatur. Der ausgefallene Dicyclohexylharnstoff wird abgesaugt und das Filtrat zur Trockne eingeengt. Die reine Titelverbindung wird durch Säulenchromatographie an Kieselgel (ca. 600 g) im Laufmittelsystem Chloroform/Methanol 5.5 : 1 erhalten.
Ausbeute : 3.0 g = 70 %

g) Tyrosyl-D-$N^\varepsilon$-formyllysyl-glycyl-phenylalanyl-homocysteinthiolacton-trifluoracetat

2 g der nach f) erhaltenen Verbindung werden mit 0,2 ml Anisol und 15 ml Trifluoressigsäure 40 Minuten bei Raumtemperatur behandelt. Man fällt durch durch Zugabe von 1 l Äther und zentrifugiert den Niederschlag. Die Substanz wird mehrfach mit Äther verrieben und der Überstand dekantiert.
Ausbeute : 1.7 g = 85 %

Beispiel 2

L-Tyrosyl-D-$N^\varepsilon$-formyllysyl-glycyl-dehydrophenylalanyl-L-prolinamid-trifluoracetat

a) Benzyloxycarbonyl-glycyl-dehydrophenylalanyl-prolinamid

11 g Benzyloxycarbonyl-glycyl-dehydrophenylalaninazlacton und 4.95 g Prolinamid-hydrochlorid werden in der erforderlichen Menge DMF gelöst und nach Zugabe von 4.4 ml N-Ethylmorpholin 24 Stunden bei Raumtemperatur belassen. Man engt ein und chromatographiert den Rückstand an einer Kieselgel-Säule (8 × 60 cm) mit dem Laufmittelgemisch $CHCl_3$-Methanol 8 : 3.
Ausbeute : 4.63 g = 31 %.
Schmp. 199-200°.

b) Glycyl-dehydrophenylalanyl-prolinamid-hydrobromid

1.3 g Benzyloxycarbonyl-glycyl-dehydrophenylalanyl-prolinamid werden in 6 ml 30 % HBr in Eisessig gelöst. Nach einer Reaktionszeit von 20 Minuten bei Raumtemperatur wird mit Äther gefällt, zentrifugiert und die Substanz mehrfach mit Äther nachgewaschen.
Ausbeute 1.14 g = 97 %
Schmp. 160° (Zers.)

c) $N^\alpha$-Benzyloxycarbonyl-D-$N^\varepsilon$-formyllysyl-glycyl-dehydrophenylalanyl-prolinamid

1.14 g Glycyl-dehydrophenylalanyl-prolinamid-hydrobromid und 1.26 g Benzyloxycarbonyl-$N^\varepsilon$-formyllysyltrichlorphenylester (Lysin-Aktivester) werden in der erforderlichen Menge DMF gelöst und nach Zugabe von 0.36 ml N-Ethyimorpholin und 387 mg 1-Hydroxybenzotriazol 18 Stunden bei Raumtemperatur gerührt. Nach Zugabe von weiteren 140 mg Lysin-Aktivester und 6 Stunden Reaktionszeit wird eingedampft, der Rückstand in Chloroform gelöst und mit 2n Citronensäure, Wasser sowie 5 % $KHCO_3$-Lösung ausgeschüttelt. Man trocknet über Natriumsulfat und engt die organische Phase zur Trockne ein.
Ausbeute 1.2 g = 70 %
Schmelzpunkt : 100° (Zers.)

d) D-$N^\varepsilon$-Formyllysyl-glycyl-dehydrophenylalanyl-prolinamid-hydrobromid

800 mg der nach d) hergestellten Benzyloxycarbonylverbindung werden in 5 ml 30 % HBr in Eisessig gelöst und 20 Minuten gerührt. Man fällt mit Äther, zentrifugiert und wäscht mehrfach mit Äther nach.
Ausbeute 700 mg = 96 %

e) tert.Butyloxycarbonyl-O-tert.butyl-tyrosyl-D-$N^\varepsilon$-formyllysyl-glycyl-dehydrophenylalanyl-prolinamid

800 mg D-$N^\varepsilon$-formyllysyl-glycyl-dehydrophenylalanylprolinamid-hydrobromid werden mit 415 mg tert.Butyloxycarbonyl-O-tert.butyl-tyrosin, 0.18 ml N-Ethylmorpholin und 915 mg 1-Hydroxybenzotriazol in 10 ml DMF gelöst. Man fügt unter Eiskühlung eine Lösung von 300 mg Dicyclohexylcarbodiimid in DMF hinzu und rührt 18 Stunden bei Raumtemperatur. Man filtriert und engt das Filtrat zur Trockne ein. Der Eindampf-Rückstand wird in Eisessig gelöst und mit jeweils 50 ml 2n Citronensäure, Wasser und 5 % Natriumbicarbonatlösung ausgeschüttelt. Der Rückstand der org. Phase wird an einer 2,5 × 40 cm Kieselgelsäule im Laufmittelsystem $CHCl_3$-Methanol-Eisessig 50 : 10 : 5 chromatographisch gereinigt.
Ausbeute 426 mg = 43 %

f) Tyrosyl-D-$N^\varepsilon$-formyllysyl-glycyl-dehydrophenylalanylprolinamid-trifluoracetat

426 mg der nach e) erhaltenen BOC-Verbindung werden in 50 µl Anisol und 5 ml Trifluoressigsäure gelöst und 30 Minuten bei Raumtemperatur gerührt. Man fällt mit 100 ml Äther und zentrifugiert.
Ausbeute 370 mg = 92 %

Schmp. 160-165° $[\alpha]_D^{22}$ + 73.6 (c = 1, Methanol)
C ber. 54.5 gef. 54.2
H ber. 5.6 gef. 5.4
N ber. 13.1 gef. 12.8

## Beispiel 3

L-Tyrosyl-D-N$^\varepsilon$-formyllysyl-glycyl-(L-threo-1.3-dihydroxy-1-phenyl)-propan-2-amid

a) tert.Butyloxycarbonyl-glycin-(L-threo-1.3-dihydro-1-phenyl)-propan-2-amid

10.6 g tert.Butyloxycarbonyl-glycin-trichlorphenylester und 5 g L(+)-threo-1-Phenyl-2-amino-propan-1.3-diol werden in 40 ml Acetonitril bis zur klaren Lösung kurz erwärmt. Man beläßt 14 Stunden bei Raumtemperatur, engt im Vakuum ein und nimmt in Essigester auf. Nach dem Ausschütteln der organischen Phase mit 2n Citronensäure und wässriger Natriumbicarbonat-Lösung wird über festem Natriumsulfat getrocknet und im Vakuum eingeengt. Es hinterbleibt ein farbloses Öl.
$R_f(CHCl_3—CH_3OH$ 8 : 3) 0.45 (Plattenmaterial : Kieselgel 60)

b) Glycin-(L-threo-1.3-dihydro-1-phenyl)-propan-2-amid-trifluoracetat

Die Gesamtmenge der nach a) erhaltenen BOC-Verbindung wird 30 Minuten mit 30 ml Trifluoressigsäure behandelt. Man engt im Vakuum ein und verreibt den Rückstand mit einem Gemisch Diisopropyläther und wasserfreiem Petroläther. Die sehr hygroskopische Substanz zerläuft an der Luft und wird über KOH im Vakuum getrocknet und aufbewahrt.
Ausbeute 10 g= 90 %

c) tert.Butyloxycarbonyl-D-N$^\varepsilon$-formyllysyl-glycin-(L-threo-1.3-dihydroxy-1-phenyl)-propan-2-amid

0.82 g Glycin-dihydroxy-phenyl-propanamid-trifluoracetat, hergestellt gemäß b), und 1.73 g tert.Butyloxycarbonyl-D-N$^\varepsilon$-formyllysin-trichlorphenylester werden unter Zusatz von 0.48 ml N-Ethylmorpholin und 0.5 g 1-Hydroxybenzotriazol in Essigester/DMF (1 : 1) zur Reaktion gebracht. Nach 60 Stunden wird im Vakuum eingeengt und das hinterbleibende Öl über eine Kieselgelsäule (2.5 × 40 cm) chromatographiert. System : Chloroform-Methanol, Gradient von 9 : 1 auf 8 : 4. Die Fraktion mit dem nachstehend angegebenen $R_f$-Wert wird eingeengt und mit Äther gefällt.
Ausbeute 1.1 g = 47 % $R_f$ = 0.6 ($CHCl_3$/MeOH/Essigsäure 50 : 10 : 5)

d) D-N$^\varepsilon$-Formyllysyl-glycin-(L-threo-1.3-dihydroxy-1-phenyl)-propan-2-amid-trifluoracetat

1.1 g BOC—D—Lys(For)—Gly—NH—CH $(CH_2OH)$—CHOH—$C_6H_5$, erhalten gemäß c), werden in 5 ml Trifluoressigsäure unter Zusatz von etwas Anisol 45 Minuten gerührt. Man engt im Vakuum etwas ein und fällt das Reaktionsprodukt mit Diethyläther.
Ausbeute 105 g = 93 %
Schmp. 98-99°, $[\alpha]_D^{22}$ = + 52.6 (C = 1, Methanol)

e) tert.Butyloxycarbonyl-tyrosyl-D-N$^\varepsilon$-formyllysyl-glycin-(L-threo-1.3-dihydroxy-1-phenyl)-propan-2-amid

1 g D—Lys(For)—Gly—NH—CH$(CH_2OH)$—CHOH—$C_6H_5 \cdot CF_3COOH$ (erhalten gemäß d)) und 0.93 tert.Butyloxycarbonyl-tyrosin-trichlorphenylester werden in DMF unter Zusatz von 0.26 ml N-Ethylmorpholin und 0.27 g 1-Hydroxybenzotriazol zur Reaktion gebracht. Nach 60 Stunden wird im Vakuum eingeengt, in Essigester aufgenommen und mit 2n Citronensäure, Wasser und Natriumbicarbonat-Lösung ausgeschüttelt.
Rohausbeute 1.2 g = 96 %
Das Rohprodukt wird über eine Kieselgelsäule (190 g $SiO_2$) mit dem Laufmittelsystem Chloroform-Methanol-Eisessig 50 : 10 : 3 chromatographiert.
Ausbeute 0.7 g = 56 %
Schmelzpunkt 96-97° (Zers.)

f) Tyrosyl-D-N$^\varepsilon$-formyllysyl-glycyl-(L-threo-1.3-dihydroxy-1-phenyl)-propan-2-amid

0.7 g BOC—Tyr—D—Lys(For)—Gly—NH—CH$(CH_2OH)$—CHOH—$C_6H_5$ werden mit 5 ml Trifluoressigsäure/Anisol (1 : 1) 45 Minuten gerührt. Man engt im Vakuum etwas ein, fällt durch Zugabe von Äther und wäscht den Niederschlag mehrfach mit Äther nach.
Ausbeute 0.5 g = 70 %
Schmelzpunkt 73-74° (Zers.) $[\alpha]_D^{22}$ = + 23.6

Aminosäure-Analyse :
Gly 1.04 Tyr 0.96 Lys 1.00 weiterer Bestandteil 1.07 (gemessen wie Phe)

**Ansprüche** (für die Vertragsstaaten : BE, CH, DE, FR, GB, IT, LI, NL, SE)

1. Neue Peptidamide der allgemeinen Formel I

H—Tyr—D—Lys(For)—Gly—X (I)

in der bedeutet
For Formyl,
X Alkylamino,
Dehydro-Phe- oder Phe-alkylamido mit bis zu 6 C-Atomen im Alkylrest, der durch Hydroxy und/oder Phenyl substituiert sein kann,
Phe-cycloalkylamid oder -cycloalkylenamid mit bis zu 8 C-Atomen im Cycloalkyl bzw. Cycloalkylen, wobei 1-2-$CH_2$-Gruppen durch —NH—, —O—, —S— oder —CO— ersetzt sein können,
Phe-alkylen-cycloalkylamid mit 5 bis 6 Ring-C-Atomen, das durch Carbonamido, N-Alkylcarbonamido oder Alkyl substituiert sein kann und in dem ein C-Atom durch N ersetzt sein kann,
Phe-endo- oder exo-norbornylamid oder Phe-thiazolamid oder -thiazolidincarbonsäureamid, das durch 1-4 Methylgruppen substituiert sein kann.

2. Verfahren zur Herstellung eines Peptidamids gemäß Anspruch 1, dadurch gekennzeichnet, daß man ein Peptidamid der Formel II

Y—Tyr—D—Lys(For)—Gly—OH (II)

in der
Y eine Schutzgruppe, vorzugsweise Benzyloxycarbonyl oder tert.Butyloxycarbonyl bedeutet, mit einem Amin XH kondensiert oder ein Peptidderivat der Formel III

Y—Tyr—D—Lys(For)—Gly—Phe—OH (III)

mit einem um den Phenylalaninrest (Phe) verkürzten Amin XH umsetzt und anschließend die Schutzgruppe Y abspaltet.

3. L-Tyrosyl-D-$N^{\varepsilon}$-formyllysyl-glycyl-L-phenylalanyl-homocysteinthiolacton.

**Ansprüche** (für den Vertragsstaat AT)

1. Verfahren zur Herstellung eines Peptidamids der allgemeinen Formel I

H—Tyr—D—Lys(For)—Gly—X (I)

in der bedeutet
For Formyl,
X Alkylamino,
Dehydro-Phe- oder Phe-alkylamido mit bis zu 6 C-Atomen im Alkylrest, der durch Hydroxy und/oder Phenyl substituiert sein kann,
Phe-cycloalkylamid oder -cycloalkylenamid mit bis zu 8 C-Atomen im Cycloalkyl bzw. Cycloalkylen, wobei 1-2-$CH_2$-Gruppen durch —NH—, —O—, —S— oder —CO— ersetzt sein können,
Phe-alkylen-cycloalkylamid mit 5 bis 6 Ring-C-Atomen, das durch Carbonamido, N-Alkylcarbonamido oder Alkyl substituiert sein kann und in dem ein C-Atom durch N ersetzt sein kann,
Phe-endo- oder exo-norbornylamid oder
Phe-thiazolamid oder -thiazolidincarbonsäureamid, das durch 1-4 Methylgruppen substituiert sein kann,
dadurch gekennzeichnet, daß man ein Peptidamid der Formel II

Y—Tyr—D—Lys(For)—Gly—OH (II)

in der
Y eine Schutzgruppe, vorzugsweise Benzyloxycarbonyl oder tert.-Butyloxycarbonyl bedeutet, mit einem Amin XH kondensiert oder ein Peptidderivat der Formel III

Y—Tyr—D—Lys(For)—Gly—Phe—OH (III)

mit einem um den Phenylalaninrest (Phe) verkürzten Amin XH umsetzt und anschließend die Schutzgruppe Y abspaltet.

2. Verfahren nach Anspruch 1. dadurch gekennzeichnet, daß man das L-Tyrosyl-D-N$^{\varepsilon}$-formyllysyl-glycyl-L-phenylalanyl-homocysteinthiolacton, herstellt.

**Claims** (for the Contracting States : BE, CH, DE, FR, GB, IT, LI, NL, SE)

1. New peptide amides of the general formula I

H—Tyr—D—Lys(For)—Gly—X (I)

wherein
For is formyl,
X is alkylamino,
dehydro-Phe or Phe-alkylamido having up to 6 carbon atoms in the alkyl moiety, which moiety may be substituted by hydroxy and/or phenyl,
Phe-cycloalkylamide or Phe-cycloalkylene amide having up to 8 carbon atoms in the cycloalkyl or cyclalkylene moiety, 1 to 2 $CH_2$ groups being optionally replaced by —NH—, —O—, —S— or —CO—,
Phe-alkylene-cycloalkylamide having from 5 to 6 ring carbon atoms, which amide may be substituted by carbonamido, N-alkylcarbonamido or alkyl and 1 carbon atom whereof may be replaced by nitrogen,
Phe-endo- or exo-norbornylamide or
Phe-thiazolamide or Phe-thiazolidine-carboxylic acid amide, which may be substituted by 1 to 4 methyl groups each.

2. A process for the manufacture of a peptide amide as claimed in claim 1, which comprises condensing a peptide amide of the formula II

Y—Tyr—D—Lys(For)—Gly—OH (II)

wherein
Y is a protective group, preferably benzyloxycarbonyl or tert. butyloxycarbonyl, with an amide XH or reacting a peptide derivative of the formula III

Y—Tyr—D—Lys(For)—Gly—Phe—OH (III)

with an amide XH, which does not contain the phenylalanine (Phe) radical and splitting off the protective group Y.

3. L-Tyrosyl-D-N$^{\varepsilon}$-formyllysyl-glycyl-L-phenylalanylhomo-cysteine-thiolactone.

**Claims** (for the Contracting State AT)

1. A process for the manufacture of a new peptide amide of the general formula I

H—Tyr—D—Lys(For)—Gly—X (I)

wherein
For is formyl,
X is alkylamino,
dehydro-Phe or Phe-alkylamido having up to 6 carbon atoms in the alkyl moiety, which moiety may be substituted by hydroxy and/or phenyl,
Phe-cycloalkylamide or Phe-cycloalkylene amide having up to 8 carbon atoms in the cycloalkyl or cyclalkylene moiety, 1 to 2 $CH_2$ groups being optionally replaced by —NH—, —O—, —S— or —CO—,
Phe-alkylene-cycloalkylamide having from 5 to 6 ring carbon atoms, which amide may be substituted by carbonamido, N-alkylcarbonamido or alkyl and 1 carbon atom whereof may be replaced by nitrogen,
Phe-endo- or exo-norbornylamide or
Phe-thiazolamide or Phe-thiazolidine-carboxylic acid amide, which may be substituted by 1 to 4 methyl groups each, which comprises condensing a peptide amide of the formula II

Y—Tyr—D—Lys(For)—Gly—OH (II)

wherein
Y is a protective group, preferably benzyloxycarbonyl or tert. butyloxycarbonyl, with an amine XH or reacting a peptide derivative of the formula III

Y—Tyr—D—Lys(For)—Gly—Phe—OH (III)

with an amine XH, which does not contain the phenylalanine (Phe) radical and splitting off the protective group Y.

2. A process according to claim 1, being a process for producing the compound L-tyrosyl-D-$N^{\varepsilon}$-formyllysylglycyl-L-phenylalanyl-homo-cysteine-thiolactone.

**Revendications** (pour les Etats contractants : BE, CH, DE, FR, GB, IT, LI, NL, SE)

1. Nouveaux amides de peptides de formule générale I

H—Tyr—D—Lys(For)—Gly—X (I)

dans laquelle

For représente un groupe formyle,

X représente un groupe alcoylamino,

dehydro-Phe- ou Phe-alcoylamido avec jusqu'à 6 atomes de carbone dans le groupe alcoyle, qui peut être substitué par un groupe hydroxy et/ou phényle,

Phe-cycloalcoylamide ou Phe-cycloalcoylène-amide avec jusqu'à 8 atomes de carbone dans le groupe cycloalcoyle ou cycloalcoylène, ou 1 ou 2 groupes —$CH_2$— peuvent être remplacés par —NH—, —O—, —S—, ou —CO—,

Phe-alcoylène-cycloalcoylamide avec de 5 à 6 atomes de carbone dans le noyau, qui peut être substitué par un groupe carbonamido, N-alcoylcarbonamido ou alcoyle et dans lequel un atome de carbone peut être remplacé par N,

Phe-endo- ou exo-norbornylamide ou

Phe-thiazolamide ou Phe-thiazolidinecarboxamide, qui peut être substitué par de 1 à 4 groupes méthyle.

2. Procédé pour la préparation d'un amide de peptide selon la revendication 1, caractérisé en ce qu'on condense un amide de peptide de formule II

Y—Tyr—D—Lys(For)—Gly—OH (II)

dans lequel

Y représente un groupe protecteur, de préférence un groupe benzyloxycarbonyle ou tert-butyloxycarbonyle, avec une amine XH ou on fait réagir un dérivé de peptide de formule III

Y—Tyr—D—Lys(For)—Gly—Phe—OH (III)

avec une amine XH diminuée du radical phénylalanine (Phe), puis on sépare le groupe protecteur Y.

3. L-tyrosyl-D-$N^{\varepsilon}$-formyllysyl-glycyl-L-phénylalanyl-homo-cystéinethiolactone.

**Revendications** (pour l'Etat contractant AT)

1. Procédé pour la préparation d'un amide de peptide de formule générale I

H—Tyr—D—Lys(For)—Gly—X (I)

dans laquelle

For représente un groupe formyle,

X représente un groupe alcoylamino,

déhydro-Phe- ou Phe-alcoylamido avec jusqu'à 6 atomes de carbone dans le groupe alcoyle, qui peut être substitué par un groupe hydroxy et/ou phényle,

Phe-cycloalcoylamide ou Phe-cycloalcoylèneamide avec jusqu'à 8 atomes de carbone dans le groupe cycloalcoyle ou cycloalcoylène, où 1 ou 2 groupes —$CH_2$— peuvent être substitués par —NH—, —O—, —S— ou —CO—,

Phe-alcoylène-cycloalcoylamide avec de 5 à 6 atomes de carbone dans le noyau, qui peut être substitué par un groupe carbonamido, N-alcoylcarbonamido ou alcoyle et dans lequel un atome de carbone peut être remplacé par N,

Phe-endo- ou exo-norbornylamide ou

Phe-thiazolamide ou -thiazolidinecarboxyamide, qui peut être substitué par de 1 à 4 groupes méthyle, caractérisé en ce qu'on condense un amide de peptide de formule II

Y—Tyr—D—Lys(For)—Gly—OH (II)

dans laquelle

Y représente un groupe protecteur, de préférence un groupe benzyloxycarbonyle ou tertbutyloxycarbonyle, avec une amine XH ou on fait réagir un dérivé de peptide de formule III

$$Y—Tyr—D—Lys(For)—Gly—Phe—OH \quad (III)$$

avec une amine XH diminuée du radical phénylalanine (Phe), puis on sépare le groupe protecteur Y.

2. Procédé selon la revendication 1, caractérisé en ce qu'on prépare la L-tyrosyl-D-$N^{\varepsilon}$-formyllysyl-glycyl-L-phénylalanyl-homo-cystéine-thiolactone.